# DEMANDE DE BREVET EUROPEEN

(11) **EP 3 200 107 A1**
(43) Date de publication de la demande: **02.08.2017**
(21) Numéro de dépôt: 16020022.6
(22) Date de dépôt: 01.02.2016
(51) Int. Cl.: G06F 19/00

(54) **PROCÉDÉ QUI ASSOCIE DES PROGRAMMES INFORMATIQUES ET LE CONDITIONNEMENT DES MÉDICAMENTS POUR AMÉLIORER L'OBSERVANCE DE TRAITEMENT MÉDICAL PAR DES PATIENTS EN SOINS AMBULATOIRES**

(71) Demandeur: Le Chevalier de Preville Yvon, 1217 Meyrin (CH)
(72) Inventeur: Le Chevalier de Preville Yvon, 1217 Meyrin (CH)

(57) **Abrégé**

Le procédé, objet de la présente invention, est basé sur un programme informatique, une base de données sécurisée, deux applications pour téléphone portable intelligent (smartphone) et un conditionnement spécial des doses d'un médicament. Il permet d'améliorer l'observance de traitement d'un médicament prescrit par un médecin.

## Description

La non-observance des ordonnances médicales par les patients ambulatoires pose de nombreux problèmes médicaux et environnementaux. On peut distinguer trois cas : les maladies chroniques, les maladies temporaires et les traitements ambulatoires préopératoires. Dans le cas des maladies chroniques, les patients ont tendance à oublier la prise de leurs médicaments, surtout s'ils doivent prendre plusieurs remèdes par jour. Il leur arrive également de prendre plus de doses d'un médicament que prescrit, car ils ont oublié s'ils ont déjà pris dans la journée. Dans le cas de maladies temporaires, les patients ont tendance à cesser la prise du médicament dès qu'ils se sentent mieux, ce qui, dans le cas de maladies d'origine microbienne engendrent des souches microbiennes résistantes. Dans le cas des traitements ambulatoires préopératoires, la prise des doses prescrites doit souvent de faire de façon précise dans le temps et le chirurgien ou médecin veulent être certains que la posologie a été suivie rigoureusement.

Il a été évalué aux États-Unis que le coût de cette non-observance pour les principales maladies chroniques (hypertension, diabètes, hypercholestérolémie, insuffisance cardiaque congestive) est d'environ 300 milliards de dollars par an. [1],[2]

Quelques inventions abordent ce problème, mais soit dans un milieu hospitalier, souvent à des fins d'études statistiques [EP2473964 A2, WO2015/191805 A1], soit dans l'optique d'adapter et contrôler la posologie [US2015356701 (A1)]. Aucun de ces brevets n'aborde le problème de l'observance des traitements de patients ambulatoires.

La présente invention est une réponse qui permettra d'améliorer l'observance des ordonnances médicales en combinant une solution technique et des incitations comportementales, à même d'amener les patients à adhérer aux ordonnances médicales, tant pour les maladies chroniques que pour les maladies temporaires, les deux traitées de manière ambulatoire.

La présente invention consiste en :
- Une application mobile, « Patient », qui permet au patient ou à son représentant légal de:
   ▪ Scanner le marquage de la boîte de médicament
   ▪ Entrer les données de l'ordonnance médicale (médecin, médicament, posologie prescrite, heures de prise, durée de la prescription,...)
   ▪ Entrer les codes de chaque dose au moment de la prise de ladite dose
   ▪ Alerter le patient de tout retard dans la prise d'une dose
   ▪ Alerter le médecin en cas de retard important dans la prise des doses
- Un programme d'inscription d'un médecin, « Gestion de l'observance « , dans la base de données de l'application mobile qui permet :
   ▪ Au médecin de fournir ses coordonnées et références
   ▪ D'encrypter ces informations pour préserver l'anonymat du médecin
   ▪ Au médecin d'être informé de l'observance de traitement de son patient
- Une application mobile, « Médecin », qui alerte le médecin en cas de non-observance par un de ses patients.
- Une base de données sécurisée contenant les informations fournies par le médecin, les informations relatives aux ordonnances et les programmes nécessaires aux communications avec les patients et les médecins.
- Le conditionnement des médicaments dans des plaquettes de telle manière que chaque dose soit dans une alvéole fermée et opaque au fond de laquelle est inscrit un code identifiant chaque dose.
- Un marquage de la boîte vendue, contenant une ou plusieurs plaquettes, marquage qui, outre les informations standards, contient un code identifiant le médicament, un code unique identifiant la boîte et les différents codes de chaque dose.

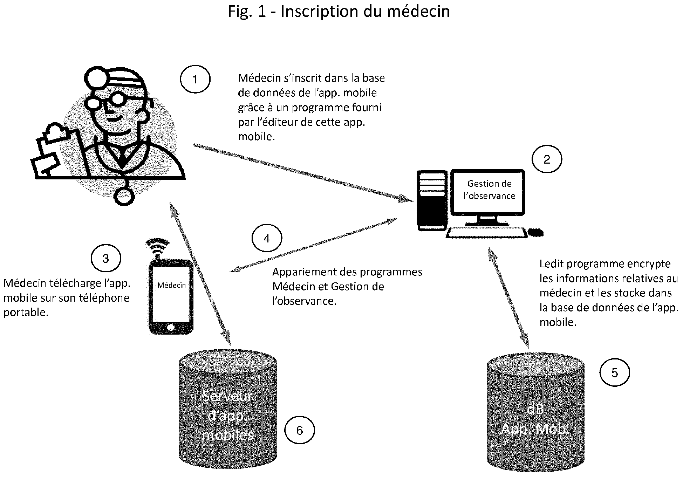

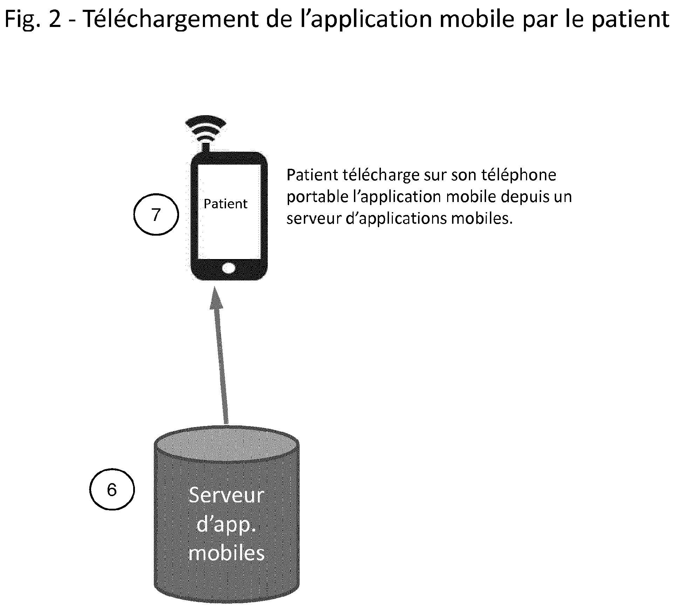

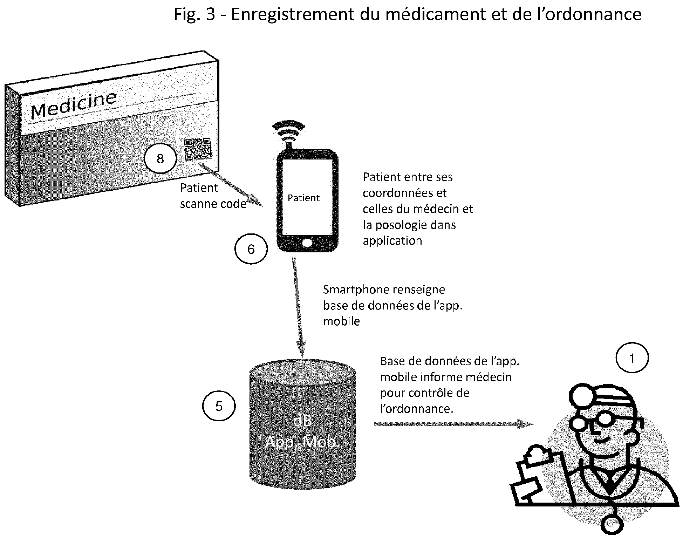

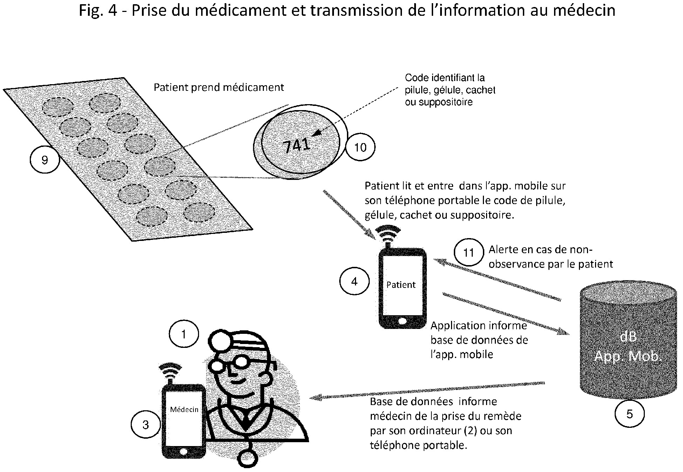

Les figures 1, 2, 3 et 4 décrivent les phases de la mise en oeuvre de l'invention par le médecin et le patient. Les différents éléments apparaissant sur ces schémas sont numérotés et ces numéros apparaissent dans la suite de ce texte en parenthèses (e.g. (5)).

Dans la suite de ce texte, le terme « patient » représente soit la personne qui doit prendre le médicament, soit la personne qui doit administrer le médicament à la personne pour laquelle le médecin a rédigé une ordonnance, par exemple un enfant, une personne handicapée ou présentant des troubles mémoriels (e.g. maladie d'Alzheimer).

La présente invention ne s'applique qu'aux médicaments délivrés sous forme de pilules, gélules, cachets, suppositoires ou liquides conditionnés pour une prise dosée. Dans la suite de ce texte, toutes ces formes de médicament seront désignées par « pilule » ou « pilules ».

### Mode opératoire.

Un médecin (1) souhaite suivre l'observance d'un médicament par ses patients. Ce médecin télécharge sur son ordinateur de bureau (2), depuis les serveurs (5) de l'éditeur des applications constituant la présente invention, une application nommée « Gestion de l'observance » et s'inscrit, au moyen de cette application, à une base de données sécurisée et cryptée, base de données gérée (5) par l'éditeur des applications mobiles, objets de la présente invention. L'application « Gestion de l'observance » cryptera les communications entre le médecin et cette base de données pour assurer l'anonymat du médecin. Il télécharge depuis un serveur d'applications mobiles (6) sur son téléphone portable intelligent (smartphone) (3) l'application mobile « Médecin », qui lui permettra d'être alerté en cas de non-observance de l'ordonnance par un de ses patients. Cette application mobile sera associée (4) à l'application « Gestion de l'observance » afin de communiquer au médecin les références des patients et médicaments prescrits par lui.

Le médecin prescrit à son patient un médicament conforme aux exigences de conditionnement de la présente invention.

Le médicament (8), pour être conforme aux exigences de conditionnement de la présente invention, doit être constitué de pilules, conditionnées dans des alvéoles individuelles opaques sur un ou plusieurs plaquettes (9). Au fond de chaque alvéole, est inscrit un code numérique (10) de 2 à 4 chiffres identifiant la pilule contenue dans ladite alvéole. Les plaquettes sont conditionnées dans une boîte, sur laquelle est apposé un code d'identification unique complexe (8), contenant un code identifiant le médicament, les codes de toutes les pilules contenues dans ladite boîte et des informations relatives à la date de péremption de ces pilules, le numéro de série de production, etc.

Le patient achète la boîte de pilules, télécharge avec son téléphone portable (7) l'application mobile nommée« Patient » depuis un serveur d'applications mobiles (6) (e.g. iTunes, Google Play, ou autres) et introduit dans cette application ses coordonnées (nom, prénom, adresse, téléphones, adresse courriel, mot de passe). Puis, grâce à la caméra de son téléphone portable, il scanne le code d'identification unique de la boîte achetée (8). L'application lui demande ensuite les références du médecin qui a prescrit le médicament et les détails de l'ordonnance que lui a prescrit le médecin : posologie prescrite, heures de prise, durée de la prescription.

L'application « Patient » alerte le patient de la prochaine prise de son médicament en indiquant le nom du médicament et la quantité à prendre. Dans le cas où le patient doit prendre au même moment plusieurs médicaments, l'application « Patient » lui indique la liste des médicaments et les quantités respectives.

Le patient ouvre la boîte du médicament, brise l'opercule qui scelle l'alvéole d'une pilule, en extrait la pilule et l'avale puis lit le code numérique inscrit au fond de l'alvéole (10) et entre ce code dans l'application « Patient ». Dès que la patient a terminé d'entrer ce code, l'application mobile « Patient » initie une communication avec la base de données (5) de l'éditeur des applications et lui signale que la pilule, ayant le code entré, a été effectivement prise par le patient. Le médecin (1) ayant prescrit le médicament pourra tirer cette base de données les données relatives aux prises de médicament par son patient.

Si le patient néglige de prendre son médicament à l'heure prescrite, l'application mobile « Patient » déclenche une alerte (11) sur le téléphone portable du patient lui rappelant le besoin de prendre son médicament. Si après un certain laps de temps (lié au médicament), le patient n'a pas entré le code de la pilule ou les codes des pilules, l'application « Patient » initie une communication avec la base de données de l'éditeur des applications qui lui signale, sur son application «Médecin » (1) (3), que le patient n'a pas pris son médicament comme prescrit. Le médecin peut alors contacter le patient ou toute autre personne qui pourra s'assurer que le patient prenne bien son médicament.

Si le patient, ayant oublié s'il a déjà pris la dose prévue pour la journée, veut en prendre une autre, l'application « Patient » lui signalera son erreur et ne lui permettra pas d'entrer le code de la pilule.

### Discussion

Le procédé, objet de la présente invention, vise à améliorer l'observance de traitement d'un médicament, tant pour une maladie chronique que pour une maladie temporaire ou un traitement préopératoire. Il ne peut toutefois pas garantir de façon absolue l'observance, qui en dernier ressort repose sur la bonne volonté du patient. En effet, si le patient n'avale pas la pilule et la jette, mais introduit le code de ladite pilule dans l'application « Patient », aucune alerte ne sera donnée. Il limite toutefois les risques de non-observance liés aux oublis ou aux prises au-delà de la prescription.

Un autre aspect de ce procédé est que le médecin ou l'application « Patient » peuvent alerter le patient de la proximité de la date de péremption d'un médicament et l'inciter à rapporter les pilules restantes à une pharmacie, au lieu de les jeter aux ordures où elles finiront pas polluer l'environnement, en particulier l'eau.
[1] IMPROVING PRESCRIPTION MEDICINE ADHERENCE IS KEYTO BETTER HEALTH CARE - Taking Medicines as Prescribed Can Lower Costs and Improve Health Outcomes- PhRMA-June 2011
[2] Impact of Medication Adherence on Hospitalization Risk and Healthcare Cost - Michael C. Sokol, MD, MS, Kimberly A. McGuigan, PhD, Robert R. Verbrugge, PhD,and Robert S. Epstein, MD, MS - Medical Care • Volume 43, Number 6, June 2005

## Revendications

1. Le procédé permettant une meilleure observance d'un traitement médical est constitué de :
• Un programme informatique, nommé « Gestion de l'observance médicale », fonctionnant avec les systèmes d'exploitation Windows, Android, OS X ou tout autre système d'exploitation d'un ordinateur de bureau, qui enregistre les données relatives à un médecin (nom, prénom, adresse postale, téléphone portable, adresse courriel, mot de passe et tout autre identification du médecin), les crypte par un algorithme de chiffrement à haute sécurité, et transmet le fichier en résultant à un serveur désigné par l'éditeur dudit programme. Ce programme permet également au médecin de télécharger de ce même serveur informatique les informations que le serveur recevra du programme « Patient » de chacun de ses patients et permet ainsi au médecin de suivre et contrôler l'observance des traitements qu'il a prescrits.
• Une application mobile, nommé « Médecin », fonctionnant avec les systèmes d'exploitation Android, iOS, Windows Phone et tout autre système d'exploitation d'un téléphone mobile ou tablette, qui enregistre les données relatives à un médecin (nom, prénom, adresse postale, téléphone portable, adresse courriel, mot de passe et tout autre identification unique du médecin), se connecte automatiquement au serveur de l'éditeur du programme « Gestion de l'observance médicale » et associe ses coordonnées (numéro d'appel, code de cryptage, mot de passe et tout autre identification du médecin) au programme « Gestion de l'observance médicale ». Ce programme envoie par voie de télécommunication au programme « Médecin » une alerte chaque fois qu'un patient n'aura pas suivi la prescription faite par ce médecin.
• Une application mobile, nommée « Patient », fonctionnant avec les systèmes d'exploitation Android, iOS, Windows Phone et tout autre système d'exploitation d'un téléphone mobile ou tablette, qui enregistre les données relatives au patient (nom, prénom, adresse postale, téléphone portable, adresse courriel, mot de passe et tout autre identification unique du patient), enregistre les données d'une prescription médicale (nom, prénom, adresse du médecin, et tout autre identification unique du médecin, le nom du médicament prescrit ou des médicaments prescrits par ce médecin ainsi que la posologie prescrite (doses, fréquence des prises du médicament, délai de retard admissible, durée de la prescription) et transmet par voie de télécommunication au serveur de l'éditeur du programme « Gestion de l'observance médicale » ces données relatives au médecin et à la prescription. Cette application mobile, grâce à la caméra du téléphone portable du patient sur lequel est installée, scanne le code d'identification unique de la boîte du médicament prescrit, enregistre la date à laquelle ce scanne est fait et transmet par voie de télécommunication au serveur de l'éditeur du programme « Gestion de l'observance médicale » ces informations relatives à cette boîte (code d'identification du médicament, code du producteur du médicament, codes d'identification de chaque pilule contenues dans ladite boîte, date de préemption, date du scanne, numéro de série de production des pilules et tout autre information relative au médicament). Cette application mobile informe le patient de la nécessité de prendre son ou ses médicaments aux moments prescrits par le médecin. Le patient, lorsqu'il prend la pilule dans son alvéole, doit entrer le code d'identification de cette pilule dans l'application mobile « Patient » qui transmet par voie de télécommunication au serveur de l'éditeur du programme « Gestion de l'observance médicale » ce code d'identification de ladite pilule. Si le patient ne fournit pas à l'application « Patient » le code de la pilule dans un délai de retard admissible, le programme « Gestion de l'observance médicale » transmet par voie de télécommunication une alerte sur le téléphone portable du médecin sur lequel est installé l'application mobile « Médecin ».
• Une identification unique par code à barres unidimensionnel ou bidimensionnel, monochrome ou polychrome de la boîte contenant les pilules du médicament qu'elle contient. Ce code d'identification unique contient le code d'identification du médicament, code du producteur du médicament, codes d'identification de chaque pilule contenue dans ladite boîte, date de préemption, numéro de série de production des pilules et tout autre information relative au médicament. Les pilules contenues dans la boîte sont conditionnées dans des alvéoles individuelles opaques sur des plaquettes. Au fond de chaque alvéole, un code identifiant la pilule contenue dans ladite alvéole est inscrit sous la forme d'un code numérique à 2, 3 ou 4 chiffres.

2. Procédé selon la revendication précédente dans lequel le téléphone portable du patient doit être muni d'un lecteur d'identification par radiofréquence (RFID) et le code d'identification unique de la boîte est un code d'identification par radiofréquence contenant les mêmes informations que dans la revendication 1. Les codes individuels des pilules restent des codes numériques à 2, 3 ou 4 chiffres.

3. Procédé selon la revendication 1 dans lequel le téléphone portable du patient doit être muni d'un lecteur d'identification par communication en champ proche (NFC) et le code d'identification unique de la boîte est un code d'identification par communication en champ proche contenant les mêmes informations que dans la revendication 1. Les codes individuels des pilules restent des codes numériques à 2, 3 ou 4 chiffres.

4. Procédé selon la revendication 1 dans lequel le téléphone portable du patient doit être muni d'un lecteur d'identification par Bluetooth et le code d'identification unique de la boîte est un code d'identification iBeacon contenant les mêmes informations que dans la revendication 1. Les codes individuels des pilules restent des codes numériques à 2, 3 ou 4 chiffres.

5. Procédé selon la revendication 2 dans lequel le téléphone portable du patient doit être muni d'un lecteur d'identification par radiofréquence (RFID) et le code d'identification unique de la boîte est un code d'identification par radiofréquence contenant les mêmes informations que dans la revendication 1. Les codes individuels des pilules sont remplacés par une identification par radiofréquence (RFID).

6. Procédé selon la revendication 2 dans lequel le téléphone portable du patient doit être muni d'un lecteur d'identification par communication en champ proche (NFC) et le code d'identification unique de la boîte est un code d'identification par communication en champ proche (NFC) contenant les mêmes informations que dans la revendication 1. Les codes individuels des pilules sont remplacés par une identification par communication en champ proche (NFC).
